# FASCICULE DE BREVET EUROPEEN

(11) **EP 3 593 824 B1**
(45) Date de publication et mention de la délivrance du brevet: **24.03.2021**
(21) Numéro de dépôt: 19182644.5
(22) Date de dépôt: 26.06.2019
(51) Int. Cl.: A61L 2/26, A61B 50/20

(54) **SYSTÈME DE SUPPORT INVISIBLE POUR INSTRUMENTS MÉDICAUX À STÉRILISER OU À NETTOYER**
UNSICHTBARES HALTERUNGSSYSTEM FÜR ZU STERILISIERENDE ODER ZU REINIGENDE MEDIZINISCHE INSTRUMENTE
INVISIBLE SUPPORT SYSTEM FOR MEDICAL INSTRUMENTS TO BE STERILISED OR CLEANED

(30) Priorité: 09.07.2018 FR 1856300
(43) Date de publication de la demande: 15.01.2020
(73) Titulaire: Applications Industrielles des Plastiques AIP, 69680 Chassieu (FR)
(72) Inventeur: BEISSMANN, Ludovic, 01300 Cuzieu (FR)
(74) Mandataire: Cabinet Beau de Loménie

(56) Documents cités:
- US-A- 5 525 314
- US-A1- 2005 115 850
- US-B2- 7 896 158

## Description

La présente invention concerne le domaine technique du nettoyage et/ou de la stérilisation d'instruments médicaux comportant, en particulier, un élément de fixation tel qu'une quille, une queue ou une tige apte à pouvoir être maintenue à travers un trou lors de l'opération de nettoyage ou de stérilisation.

L'objet de l'invention vise plus précisément les équipements de conditionnement pour la stérilisation ou le nettoyage d'instruments médicaux comme par exemple les implants en particuliers dentaires ou orthopédiques, et les instruments chirurgicaux.

Dans le domaine d'application préféré de l'invention, il apparait le besoin de pouvoir nettoyer et/ou stériliser de tels instruments médicaux. A cet effet, les instruments médicaux sont maintenus classiquement à l'aide d'un système de support permettant la manutention et le positionnement des instruments médicaux à l'intérieur des machines à stériliser et/ou à nettoyer.

Dans l'état de la technique, il est connu différents systèmes de support tels que des plateaux, des conteneurs ou des boîtes, réalisés en matière plastique ou métallique. Un système de support comporte généralement une plaque présentant une face interne et une face externe, et dans laquelle est aménagée, classiquement, une série de trous adaptés chacun pour recevoir un élément de fixation d'un instrument médical introduit à partir de la face externe de la plaque. Chaque instrument médical est ainsi maintenu en position lors des diverses opérations de manutention ayant pour objectif leur nettoyage ou stérilisation mais également pendant l'opération proprement dite de nettoyage ou de stérilisation. Un tel système de support doit garantir un maintien des instruments médicaux lors de ce processus de nettoyage ou de stérilisation tout en limitant les surfaces de contact avec l'instrument afin de garantir un bon nettoyage ou stérilisation. Un tel système de support doit être conçu également de manière à limiter les contaminations lors des opérations de manipulation des instruments médicaux afin de faciliter les opérations de nettoyage et de stérilisation.

Il est connu dans l'état de la technique, de nombreuses solutions pour maintenir les instruments médicaux lors de ce processus de nettoyage et garantir un bon nettoyage ou stérilisation. Une solution consiste à équiper chaque trou de la plaque support, d'un œillet réalisé en un matériau souple ou flexible, pour retenir par friction, l'instrument médical. Dans le cas où les œillets restent montés de façon permanente à la plaque support, il apparaît une difficulté à obtenir un nettoyage ou une stérilisation efficace du système de support, en particulier au niveau de la jonction entre l'œillet et la plaque support.

Afin de garantir un bon nettoyage ou une stérilisation efficace, les documents WO 2013/040363 et WO 2013/040380 proposent de munir chaque œillet, d'un système de liaison démontable avec la plaque. D'une manière générale, chaque œillet comporte un fût tubulaire pourvu à une extrémité, d'un rebord prenant appui sur la face externe de la plaque et à son autre extrémité, d'un bourrelet venant se bloquer sur la face interne de la plaque. En pratique, les opérations de montage et démontage conduisent à une dégradation de l'œillet susceptible de créer des zones difficiles à nettoyer ou à stériliser.

La demande de brevet WO 2012/084199 décrit une cassette pour maintenir des instruments médicaux comportant une plaque pourvue de trous et une structure de maintien élastique montée sur la plaque par une liaison par encliquetage. Cette structure de maintien élastique est montée pour être située à distance de la surface supérieure de la plaque et de manière que le logement de maintien d'un instrument se trouve formé en partie haute, par le trou de la plaque et en partie basse, par un alésage aménagé dans la structure de maintien élastique. Un inconvénient de ce système concerne la difficulté à obtenir un nettoyage ou une stérilisation efficace en particulier au niveau de l'interface entre la plaque et la structure de maintien élastique.

Il est également connu notamment par le document WO 2014/089420, de réaliser le système de support par une feuille en matière souple interposée entre une plaque et une contre plaque assemblées ensemble. La plaque et la contre plaque ainsi que la feuille en matière souple sont pourvues de trous aménagés en coïncidence pour permettre le maintien des instruments médicaux. Cette solution présente un coût non négligeable en raison de l'utilisation d'une feuille en matière souple.

Le brevet US 5 525 314 décrit un système de support d'instruments médicaux à l'aide d'œillets comportant chacun une rainure annulaire aménagée sur sa surface latérale externe, avec une épaisseur comparable à celle de la plaque de fixation. Cet œillet comporte de part et d'autre de la rainure annulaire, des rebords de montage venant coopérer avec la face supérieure et la face inférieure de la plaque. L'œillet présente un passage traversant comportant une surface latérale interne pourvue d'excroissances. Un tel système de support présente une difficulté à être nettoyé ou stérilisé.

La présente invention vise à remédier aux inconvénients de l'état de la technique en proposant un nouveau système de support pour instruments médicaux à stériliser et/ou à nettoyer, conçu pour éviter d'être contaminé, pour améliorer le nettoyage et/ou la stérilisation des instruments médicaux tout en étant de conception simple en présentant un coût réduit.

Pour atteindre un tel objectif, le dispositif de support d'instruments médicaux à stériliser et/ou à nettoyer comportant chacun un élément de fixation comporte une plaque présentant une face plane inférieure et une face plane supérieure et dans laquelle est aménagé au moins un trou traversant de montage pour un œillet présentant dans sa partie centrale, un unique passage de réception et de maintien par coincement, d'un élément de fixation d'un instrument médical, cet oeillet présentant une surface supérieure s'étendant en retrait par rapport à la face supérieure de la plaque et une surface latérale externe pourvue d'un système de liaison démontable avec la plaque.

Selon l'invention, l'œillet présente une surface inférieure s'étendant en retrait de la face plane inférieure de la plaque et le passage de l'œillet est délimité par au moins trois excroissances espacées selon la circonférence de la surface intérieure de l'œillet et s'étendant radialement en saillie à partir de la surface intérieure de l'œillet, la surface intérieure de l'œillet présentant une section supérieure à la section du trou prise entre la face supérieure de la plaque et l'œillet et à la section du trou prise entre la face inférieure de la plaque et l'œillet de sorte que seules les excroissance sont visibles à travers le trou.

L'œillet selon l'invention se retrouve ainsi monté à l'intérieur de la plaque en restant visible à travers le trou aménagé dans la plaque de support, uniquement par les excroissances. L'œillet qui est ainsi caché par rapport à l'environnement extérieur peut difficilement être contaminé ou souillé. Il en ressort une facilité pour obtenir son nettoyage et/ou sa stérilisation.

De plus, le système selon l'invention peut comporter en outre en combinaison au moins l'une et/ou l'autre des caractéristiques additionnelles suivantes :
- l'œillet comporte entre trois et huit excroissances et de préférence entre trois et cinq excroissances ;
- l'œillet présente des excroissances chacune en forme d'arc de cercle ;
- le trou traversant présente entre la surface supérieure de l'œillet et la face plane supérieure de plaque, une section inférieure de celle prise entre la surface inférieure de l'œillet et la face plane inférieure de la plaque ;
- l'œillet comporte, sur sa surface latérale externe en tant que système de liaison démontable, une nervure annulaire coopérant avec un logement de forme complémentaire aménagé dans la paroi intérieure du trou de montage ;
- l'œillet comporte, sur sa surface latérale externe en tant que système de liaison démontable, une portion tronconique ;
- l'œillet comporte, sur sa surface latérale externe en tant que système de liaison démontable, un filet de pas de vis coopérant avec un taraudage du trou traversant.

Un autre objet de l'invention est de proposer un équipement de conditionnement pour la stérilisation et/ou le nettoyage d'instruments médicaux à éléments de fixation comportant au moins un dispositif de support conforme à l'invention.

Diverses autres caractéristiques ressortent de la description faite ci-dessous en référence aux dessins annexés qui montrent, à titre d'exemples non limitatifs, des formes de réalisation de l'objet de l'invention.
La **Figure 1** est une vue de ¾ de dessus en perspective montrant un exemple de réalisation du système de support conforme à l'invention, visant à maintenir en position des instruments médicaux.
La **Figure 2** est une vue de dessus du système de support illustré à la **Fig. 1****.**
La **Figure 3** est une vue en coupe du système de support prise selon les lignes III-III de la **Fig. 2** avec une vue à grande échelle.
Les **Figures 4A** et **4B** sont des vues respectivement de dessus et en perspective montrant un exemple de réalisation d'un œillet de maintien des instruments médicaux avant son montage sur une plaque.
La **Figure 5** est une vue en coupe, montrant le système de support conforme à l'invention maintenant des instruments médicaux avec une vue à plus grande échelle.
La **Figure 6** est une vue de dessous du système de support illustré à la **Fig. 1****.**

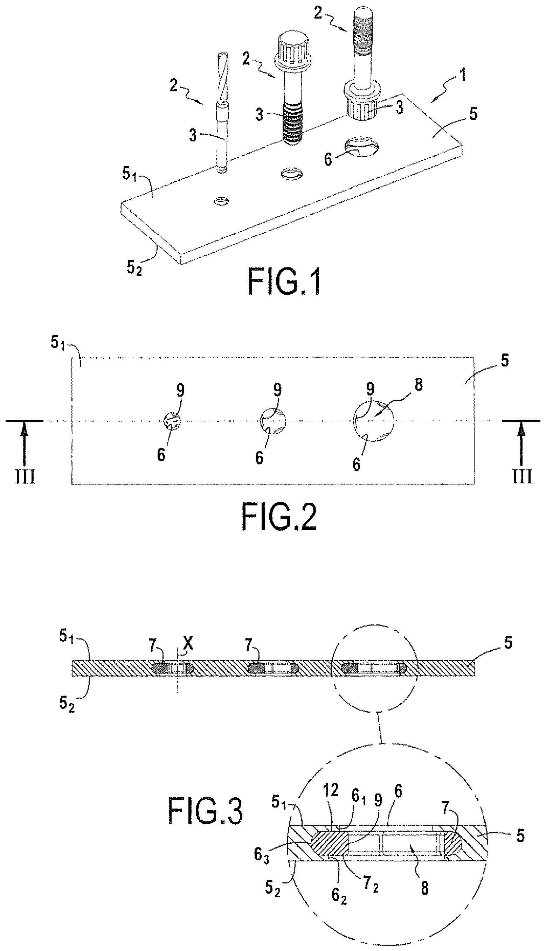

Tel que cela ressort plus précisément des **Fig. 1** à **6****,** l'objet de l'invention concerne un dispositif de support **1** pour des instruments médicaux **2** à nettoyer et/ou à stériliser de tout type connu en soi. D'une manière générale, chaque instrument médical **2** comporte un élément de fixation **3** tel qu'une quille, une queue ou une tige apte à pouvoir être maintenue à travers un trou lors de l'opération de nettoyage ou de stérilisation. Les instruments médicaux **2** sont par exemple des implants en particuliers dentaires ou orthopédiques ou des instruments chirurgicaux. Il est à noter que les instruments médicaux **2** ont été représentés sur les dessins essentiellement à titre d'illustration non limitative.

Le dispositif de support **1** selon l'invention comporte une plaque **5** présentant au moins un, et dans l'exemple illustré, plusieurs trous **6** aménagés chacun pour assurer le montage d'une structure souple ou flexible **7** de maintien pour l'élément de fixation **3** d'un instrument médical. Tel que cela des dessins, la structure souple ou flexible **7** se présente sous la forme d'un œillet présentant dans sa partie centrale, un unique passage **8** de réception et de maintien par coincement, d'un élément de fixation **3** d'un instrument médical.

La plaque **5** présente une première face dite externe ou supérieure **5₁** et une deuxième face dite interne ou inférieure **5₂**, prises en considération du sens de positionnement de l'instrument médical **3** par rapport à la plaque. Ainsi, il est à considérer que l'élément de fixation **3** d'un instrument médical est maintenu par un œillet **7** en étant introduit (mais aussi retiré) à partir de la face supérieure **5₁** de la plaque.

Chaque trou **6** aménagé dans la plaque **5** est traversant c'est-à-dire qu'il débouche sur la face supérieure **5₁** et la face inférieure **5₂**. Le trou **6** débouche sur la face supérieure **5₁** par une paroi intérieure **6₁** dite supérieure et débouche sur la face inférieure **5₂** par une paroi intérieure **6₂** dite inférieure. Chaque trou **6** présente un axe longitudinal de symétrie **X** sensiblement perpendiculaire à la face supérieure **5₁** et la face inférieure **5₂**.

La plaque **5** est réalisée en tout matériau approprié apte à supporter une opération de nettoyage et/ou de stérilisation. Par exemple, la plaque **5** est réalisée en matière plastique ou en matériau métallique. Dans l'exemple illustré, la plaque **5** présente des trous **6** aménagés selon une seule rangée mais il est clair que d'une manière générale, la plaque présente une série de trous pour la réception et le support de plusieurs instruments médicaux, répartis de toute manière appropriée selon la surface de la plaque. Le dispositif de support **1** se présente donc sous la forme d'une plaque avec une forme et des dimensions adaptées aux applications visées. Il est à noter que la plaque **5** peut être aménagée pour recevoir des systèmes de support différents de l'objet de l'invention pour le maintien d'instruments médicaux complémentaires de ceux maintenus par un œillet **7.** D'une manière générale, le dispositif de support **1** fait partie d'un équipement de conditionnement pour la stérilisation et le nettoyage d'instruments médicaux se présentant sous la forme d'une plaque ou de plateaux, intégrés ou non à l'intérieur de boîtes ou de kits de nettoyage ou de stérilisation.

L'œillet **7** est réalisé en un matériau souple ou flexible c'est-à-dire présentant une capacité à se déformer élastiquement, de manière réversible. Typiquement, l'œillet **7** est réalisé en tous matériaux appropriés adaptés pour supporter une opération de nettoyage et/ou de stérilisation.

De manière classique, l'œillet **7** se présente sous la forme d'une bague ou d'un corps annulaire aplati, avec une épaisseur délimitée entre une surface supérieure **7₁** et une surface inférieure **7₂**. La surface supérieure **7₁** et la surface inférieure **7₂** sont planes et s'étendent sensiblement parallèlement entre-elles.

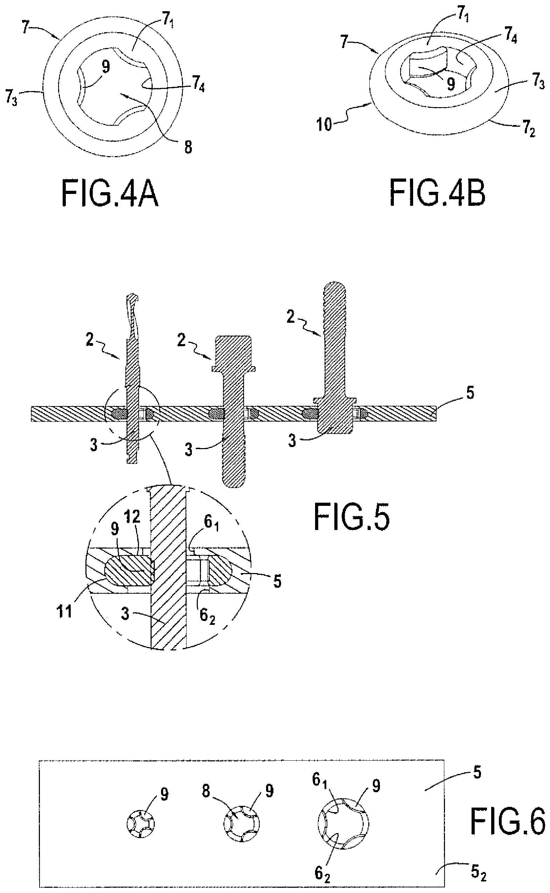

Selon une caractéristique de l'invention, l'œillet **7** présente une surface supérieure **7₁** s'étendant en retrait par rapport à la face supérieure **5₁** de la plaque **5**, comme cela ressort plus précisément des **Fig. 3** et **5****.** En d'autres termes, la surface supérieure **7₁** de l'œillet **7** est séparée de la face supérieure **5₁** de la plaque, par la paroi intérieure **6₁** du trou possédant une hauteur de l'ordre par exemple de 1/5 de l'épaisseur de la plaque.

Selon une caractéristique avantageuse de réalisation, l'œillet **7** présente une surface inférieure **7₂** s'étendant en retrait de la face plane inférieure **5₂** de la plaque. En d'autres termes, la surface inférieure **7₂** de l'œillet **7** est séparée de la face inférieure **5₂** de la plaque, par la paroi intérieure **6₂** du trou possédant une hauteur de l'ordre par exemple de 1/5 de l'épaisseur de la plaque.

L'œillet **7** comporte entre la surface supérieure **7₁** et la surface inférieure **7₂**, une surface latérale externe **7₃** et une surface intérieure **7₄**. Conformément à l'invention, l'œillet **7** est pourvu à partir de sa surface intérieure **7₄**, d'au moins trois excroissances **9** s'étendant radialement en saillie à partir de la surface intérieure de l'œillet Ces excroissances **9** délimitent le passage ou le trou **8** débouchant sur la surface supérieure **7₁** et la surface inférieure **7₂** en présentant une configuration adaptée pour maintenir un élément de fixation **3,** par coincement en raison du caractère souple ou déformable des excroissances **9.**

Le passage **8** de l'œillet **7** est délimité par au moins trois excroissances **9** espacées selon la circonférence de la surface intérieure **7₄** de l'œillet. Tel que cela ressort des **Fig. 4A** et **4B**, les excroissances **9** sont espacées sur la circonférence de la surface intérieure **7₄** de l'œillet et de préférence espacées régulièrement selon la circonférence de cette surface intérieure. Par exemple, l'œillet **7** comporte entre trois et huit excroissances **9** et de préférence entre trois et cinq excroissances.

Selon une variante préférée de réalisation, chaque excroissance **9** présente une forme en arc de cercle. Selon cette forme de réalisation, les excroissances **9** sont toutes tangentes en des points distribués sur un cercle délimitant le passage **8** de l'œillet. Il s'ensuit que l'élément de fixation **3** d'un instrument médical se retrouve en contact ponctuel ou quasi ponctuel avec chacune des excroissances **9.**

Avantageusement, les excroissances **9** de l'œillet sont espacées deux à deux d'une mesure inférieure au diamètre de l'élément de fixation **3** d'un instrument médical. Une telle caractéristique permet d'obtenir un maintien sûr de l'élément de fixation par les excroissances **9**.

Selon une caractéristique avantageuse de l'invention, la surface intérieure **7₄** de l'œillet **7** présente une section supérieure à la section du trou **6** prise entre la face supérieure **5₁** de la plaque et l'œillet **7.** En d'autres termes, la paroi intérieure **6₁** du trou délimitée entre la face supérieure **5₁** de la plaque et l'œillet **7** possède une section inférieure à la section délimitée par la surface intérieure **7₄** de l'œillet **7.** Il s'ensuit qu'à partir de la face supérieure **5₁** de la plaque, seules les excroissances **9** sont visibles à travers le trou **6.** Ainsi, tel que cela ressort de la **Fig. 2****,** seules les trois excroissances **9** sont visibles à travers le trou **6** à partir de la face supérieure **5₁**.

Selon une autre caractéristique avantageuse de l'invention, la surface intérieure **7₄** de l'œillet **7** présente une section supérieure à la section du trou **6** prise entre la face inférieure **5₂** de la plaque et l'œillet **7.** En d'autres termes, la paroi intérieure **6₂** du trou délimitée entre la face inférieure **5₂** de la plaque et l'œillet **7** possède une section inférieure à la section délimitée par la surface intérieure **7₄** de l'œillet **7.** Il s'ensuit qu'à partir de la face inférieure **5₂** de la plaque, seules les excroissances **9** sont visibles à travers le trou **6** comme illustré à la **Fig. 6****.**

Selon une autre caractéristique de l'invention, le trou traversant **6** présente entre la surface supérieure **7₁** de l'œillet et la face plane supérieure **5₁** de plaque, une section inférieure de celle prise entre la surface inférieure **7₂** de l'œillet et la face plane inférieure **5₂** de la plaque. Ainsi, la paroi intérieure supérieure **6₁** possède une section inférieure à la section de la paroi intérieure inférieure **6₂**.

Chaque œillet **7** présente une surface latérale externe **7₃** qui est aménagée pour présenter un système de liaison démontable **10.**

En d'autres termes, ce système **10** assure une liaison ou un assemblage démontable entre l'œillet **7** et la plaque **5.** Cet œillet **7** qui présente un caractère amovible, peut ainsi être fixé de manière temporaire à la plaque **5** et être démonté à volonté sans entrainer une détérioration de l'œillet ni de la plaque. Typiquement, l'œillet **7** est monté sur la plaque **5** selon au moins un sens de montage à partir d'une face, de préférence, la face inférieure **5₂** de la plaque. Bien entendu, l'œillet **7** peut être monté sur la plaque **5** selon deux sens de montage à savoir indifféremment à partir de la face supérieure **5₁** ou de la face inférieure **5₂**.

Chaque trou de montage **6** comporte un système de liaison démontable **11** qui est complémentaire du système de liaison démontable **10** de l'œillet **7,** ce système de liaison démontable complémentaire **11** étant aménagé exclusivement à partir de la paroi intérieure **6₁** du trou de montage **6.** En d'autres termes, ce système de liaison démontable complémentaire **11** prend place exclusivement sur la paroi intérieure du trou, en n'étant pas réalisé sur la face supérieure **5₁** et ni sur la face inférieure **5₂** de la plaque **5.** Il doit être compris qu'un œillet **7** et la paroi intérieure d'un trou de montage sont pourvues des systèmes de liaison démontables **10**, **11** dont l'un est complémentaire de l'autre. Ces systèmes de liaison démontable **10, 11** sont complémentaires entre eux dans le sens où ils coopèrent entre eux avec des formes géométriques présentant une congruence ou une complémentarité assurant un assemblage amovible entre l'œillet **7** et la plaque **5.** Réciproquement, le système de liaison démontable **10** de l'œillet **7** est aménagé exclusivement à partir de la surface latérale externe **7₃** de l'œillet.

Le système de liaison démontable complémentaire **11** comporte au moins une surface d'arrêt **12** de l'œillet **7,** selon au moins un sens de montage de l'œillet **7.** Cette surface d'arrêt **12** qui est réalisée à partir de la paroi intérieure du trou, limite ainsi l'introduction de l'œillet **7** à l'intérieur de la paroi intérieure **6₁**, **6₂** du trou de montage, à partir d'une face et de préférence, de la face inférieure **5₂** de la plaque.

De préférence, le système de liaison démontable complémentaire **11** comporte deux surfaces d'arrêt **12** pour l'œillet **7,** selon les deux sens de montage de l'œillet sur la plaque.

Chaque surface d'arrêt **12** constitue ainsi une surface de blocage pour l'œillet **7,** selon l'un ou les deux sens de la direction perpendiculaire aux faces de la plaque c'est-à-dire parallèle à l'axe longitudinal de symétrie **X** du trou **6.**

Selon la variante de réalisation illustrée sur les dessins, l'œillet **7** comporte, sur sa surface latérale externe **7₃** en tant que système de liaison démontable **10,** une nervure annulaire coopérant avec un logement **6₃** de forme complémentaire aménagé dans la paroi intérieure du trou de montage **6.** Par exemple, l'œillet **7** comporte une nervure annulaire de section droite demi-circulaire s'étendant en saillie à partir de la surface latérale externe **7₃** de l'œillet et engagée à l'intérieur d'un logement ou d'une rainure annulaire **6₃** de section droite demi-circulaire. Selon cet exemple, cette rainure annulaire **6₃** forme le système de liaison démontable complémentaire **11** et présente deux surfaces d'arrêt **12** pour l'œillet coopérant avec les surfaces supérieure **7₁** et inférieure **7₂**. Ainsi, le montage de l'œillet **7** sur la plaque **5** est réalisé par emboîtement. Bien entendu, la nervure est aménagée dans la paroi intérieure du trou de montage **6**, se prolongeant de part et d'autre par la paroi intérieure dite supérieure **6₁** et la partie intérieure dite inférieure **6₂**.

Selon une autre variante de réalisation, l'œillet **7** comporte, sur sa surface latérale externe **7₃** en tant que système de liaison démontable **10,** une portion tronconique, un filet de pas de vis ou un ergot coopérant respectivement en tant que système de liaison démontable complémentaire **11,** avec un logement tronconique, un taraudage ou une lumière aménagée à partir de la paroi intérieure du trou **6.**

Le dispositif de support selon l'invention présente l'avantage de ne pas présenter de zones difficiles à nettoyer ou à stériliser. En effet, l'œillet **7** peut difficilement être souillé ou contaminé car il se retrouve encastré à l'intérieur de la plaque en retrait de la face supérieure **5₁** de la plaque et de la face inférieure **5₂**. Seules les excroissances **9** sont visibles à partir des faces de la plaque de montage. De plus, l'œillet **7** de maintien des instruments médicaux est démontable, ce qui permet de pouvoir changer ces œillets tout en autorisant un nettoyage ou stérilisation efficace du dispositif de support selon l'invention.

## Revendications

1. Dispositif de support d'instruments médicaux **(2)** à stériliser et/ou à nettoyer comportant chacun un élément de fixation **(3),** le dispositif de support **(1)** comportant une plaque **(5)** présentant une face plane inférieure (**5₂**) et une face plane supérieure (**5₁**) et dans laquelle est aménagé au moins un trou traversant **(6)** de montage pour un œillet **(7)** présentant dans sa partie centrale, un unique passage **(8)** de réception et de maintien par coincement, d'un élément de fixation **(3)** d'un instrument médical, cet œillet **(7)** présentant une surface supérieure (**7₁**) s'étendant en retrait par rapport à la face supérieure (**5₁**) de la plaque **(5)** et une surface latérale externe (**7₃**) pourvue d'un système de liaison démontable **(10)** avec la plaque **(5), caractérisé en ce que** l'œillet **(7)** présente une surface inférieure (**7₂**) s'étendant en retrait de la face plane inférieure (**5₂**) de la plaque et **en ce que** le passage **(8)** de l'œillet **(7)** est délimité par au moins trois excroissances **(9)** espacées selon la circonférence de la surface intérieure de l'œillet et s'étendant radialement en saillie à partir de la surface intérieure de l'œillet, la surface intérieure de l'œillet présentant une section supérieure à la section du trou **(6)** prise entre la face supérieure de la plaque et l'œillet et à la section du trou prise entre la face inférieure de la plaque et l'œillet de sorte que seules les excroissances sont visibles à travers le trou **(5).**

2. Dispositif de support selon la revendication précédente, **caractérisé en ce que** l'œillet **(7)** comporte entre trois et huit excroissances et de préférence entre trois et cinq excroissances.

3. Dispositif de support selon l'une des revendications précédentes, **caractérisé en ce que** l'œillet **(7)** présente des excroissances chacune en forme d'arc de cercle.

4. Dispositif de support selon l'une des revendications précédentes, **caractérisé en ce que** le trou traversant **(6)** présente entre la surface supérieure (**7₁**) de l'œillet et la face plane supérieure (**5₁**) de plaque, une section inférieure de celle prise entre la surface inférieure (**7₂**) de l'œillet et la face plane inférieure (**5₂**) de la plaque.

5. Dispositif de support selon l'une des revendications précédentes, **caractérisé en ce que** l'œillet **(7)** comporte, sur sa surface latérale externe (**7₃**) en tant que système de liaison démontable **(10),** une nervure annulaire coopérant avec un logement de forme complémentaire aménagé dans la paroi intérieure **(6₁)** du trou de montage.

6. Dispositif de support selon l'une des revendications 1 à 4, **caractérisé en ce que** l'œillet **(7)** comporte, sur sa surface latérale externe (**7₃**) en tant que système de liaison démontable **(10),** une portion tronconique.

7. Dispositif de support selon l'une des revendications 1 à 4, **caractérisé en ce que** l'œillet **(7)** comporte, sur sa surface latérale externe (**7₃**) en tant que système de liaison démontable **(10),** un filet de pas de vis coopérant avec un taraudage du trou traversant **(6).**

8. Equipement de conditionnement pour la stérilisation et/ou le nettoyage d'instruments médicaux **(2)** à éléments de fixation **(3), caractérisé en ce qu'**il comporte au moins un dispositif de support **(1)** conforme à l'une des revendications 1 à 7.

9. Equipement selon la revendication précédente, selon lequel le dispositif de support (1) maintient au moins un instrument médical **(2)** comportant un élément de fixation **(3),** les excroissances **(9)** de l'œillet étant espacées deux à deux d'une mesure inférieure au diamètre de l'élément de fixation **(3)** de l'instrument médical.

## Patentansprüche

1. Halterungsvorrichtung für zu sterilisierende und/oder zu reinigende medizinische Instrumente (2), die jeweils ein Befestigungselement (3) beinhalten, wobei die Halterungsvorrichtung (1) eine Platte (5) beinhaltet, die eine untere ebene Fläche (5₂) und eine obere ebene Fläche (5₁) aufweist, und in der zumindest ein durchgehendes Montageloch (6) für eine Öse (7) vorgesehen ist, die in ihrem mittigen Teil einen einmaligen Durchlass (8) zur Aufnahme und zum Halten eines Befestigungselements (3) eines medizinischen Instruments durch Klemmung aufweist, wobei diese Öse (7) eine obere Oberfläche (7₁), die sich zurückgesetzt in Bezug auf die obere Fläche (5₁) der Platte (5) erstreckt, und eine äußere seitliche Oberfläche (7₃) aufweist, die mit einem lösbaren Verbindungssystem (10) mit der Platte (5) ausgestattet ist, **dadurch gekennzeichnet, dass** die Öse (7) eine untere Oberfläche (7₂) aufweist, die sich zurückgesetzt zu der unteren ebenen Fläche (5₂) der Platte erstreckt, und dass der Durchlass (8) der Öse (7) durch zumindest drei Vorsprünge (9) begrenzt ist, die entlang des Umfangs der unteren Oberfläche der Öse beabstandet sind und sich radial vorspringend ab der unteren Oberfläche der Öse erstrecken, wobei die untere Oberfläche der Öse eine Sektion über der Sektion des Lochs (6) zwischen der oberen Fläche der Platte und der Öse und der Sektion des Lochs zwischen der unteren Fläche der Platte und der Öse aufweist, sodass lediglich die Vorsprünge durch das Loch (5) sichtbar sind.

2. Halterungsvorrichtung nach dem vorhergehenden Anspruch, **dadurch gekennzeichnet, dass** die Öse (7) zwischen drei und acht Vorsprünge und bevorzugt zwischen drei und fünf Vorsprünge beinhaltet.

3. Halterungsvorrichtung nach einem der vorhergehenden Ansprüche, **dadurch gekennzeichnet, dass** die Öse (7) Vorsprünge aufweist, die jeweils in der Form eines Kreisbogens sind.

4. Halterungsvorrichtung nach einem der vorhergehenden Ansprüche, **dadurch gekennzeichnet, dass** das durchgehende Loch (6) zwischen der oberen Oberfläche (7₁) der Öse und der oberen ebenen Fläche (5₁) der Platte eine Sektion unter jener zwischen der unteren Oberfläche (7₂) der Öse und der unteren ebenen Fläche (5₂) der Platte aufweist.

5. Halterungsvorrichtung nach einem der vorhergehenden Ansprüche, **dadurch gekennzeichnet, dass** die Öse (7) auf ihrer äußeren seitlichen Oberfläche (7₃) als lösbares Verbindungssystem (10) eine ringförmige Rippe beinhaltet, die mit einer Aufnahme in komplementärer Form kooperiert, die in der Innenwand (6₁) des Montagelochs vorgesehen ist.

6. Halterungsvorrichtung nach einem der Ansprüche 1 bis 4, **dadurch gekennzeichnet, dass** die Öse (7) auf ihrer äußeren seitlichen Oberfläche (7₃) als lösbares Verbindungssystem (10) einen kegelstumpfförmigen Teil beinhaltet.

7. Halterungsvorrichtung nach einem der Ansprüche 1 bis 4, **dadurch gekennzeichnet, dass** die Öse (7) auf ihrer äußeren seitlichen Oberfläche (7₃) als lösbares Verbindungssystem (10) ein Gewinde für ein Schraubengewinde beinhaltet, das mit einem Innengewinde des durchgehenden Lochs (6) kooperiert.

8. Verpackungsausrüstung zur Sterilisation und/oder Reinigung von medizinischen Instrumenten (2) mit Befestigungselementen (3), **dadurch gekennzeichnet, dass** sie zumindest eine Halterungsvorrichtung (1) nach einem der Ansprüche 1 bis 7 beinhaltet.

9. Ausrüstung nach dem vorhergehenden Anspruch, wobei die Halterungsvorrichtung (1) zumindest ein medizinisches Instrument (2) hält, das ein Befestigungselement (3) beinhaltet, wobei die Vorsprünge (9) der Öse paarweise von einer kleineren Messung als dem Durchmesser des Befestigungselements (3) des medizinischen Elements beabstandet sind.

## Claims

1. A support device for medical instruments **(2)** to be sterilized and/or cleaned, each including a fixing element **(3),** the support device **(1)** including a plate **(5)** having a lower planar face (**5₂**) and a upper planar face (**5₁**) and in which is formed at least one mounting through hole **(6)** for an eyelet **(7)** having, in its central part, a single passage **(8)** for receiving and holding, by wedging, a fixing element **(3)** of a medical instrument, this eyelet **(7)** having an upper surface (**7₁**) extending set back from the upper face (**5₁**) of the plate **(5)** and an outer lateral surface (**7₃**) provided with a system for demountable connection **(10)** with the plate **(5), characterized in that** the eyelet **(7)** has a lower surface (**7₂**) extending set back from the lower planar face (**5₂**) of the plate and **in that** the passage **(8)** of the eyelet **(7)** is delimited by at least three protrusions **(9)** spaced apart along the circumference of the internal surface of the eyelet and radially protruding from the internal surface of the eyelet, the internal surface of the eyelet having a section greater than the section of the hole **(6)** taken between the upper face of the plate and the eyelet and than the section of the hole taken between the lower face of the plate and the eyelet so that only the protrusions are visible through the hole **(5).**

2. The support device according to the preceding claim, **characterized in that** the eyelet **(7)** includes between three and eight protrusions and preferably between three and five protrusions.

3. The support device according to any of the preceding claims, **characterized in that** the eyelet **(7)** has protrusions each in the form of a circular arc.

4. The support device according to any of the preceding claims, **characterized in that** the through hole **(6)** has between the upper surface (**7₁**) of the eyelet and the upper planar face (**5₁**) of the plate, a lower section of the one taken between the lower surface (**7₂**) of the eyelet and the lower planar face (**5₂**) of the plate.

5. The support device according to any of the preceding claims, **characterized in that** the eyelet **(7)** includes, on its outer lateral surface (**7₃**) as a demountable connection system **(10),** an annular rib cooperating with a complementary shaped housing formed in the internal wall **(6₁)** of the mounting hole.

6. The support device according to any of claims 1 to 4, **characterized in that** the eyelet **(7)** includes, on its outer lateral surface (**7₃**) as a demountable connection system **(10),** a frustoconical portion.

7. The support device according to any of claims 1 to 4, **characterized in that** the eyelet **(7)** includes, on its outer lateral surface (**7₃**) as a demountable connection system **(10),** a screw pitch thread cooperating with a tapping of the through hole **(6).**

8. Packaging equipment for the sterilization and/or cleaning of medical instruments **(2)** with fixing elements **(3), characterized in that** it includes at least one support device **(1)** according to any of claims 1 to 7.

9. Equipment according to the preceding claim, wherein the support device **(1)** holds at least one medical instrument **(2)** including a fixing element **(3),** the protrusions **(9)** of the eyelet being spaced apart two by two by a measurement smaller than the diameter of the fixing element **(3)** of the medical instrument.
